# EUROPEAN PATENT APPLICATION

(11) **EP 3 406 184 A1**
(43) Date of publication of application: **28.11.2018**
(21) Application number: 17741408.3
(22) Date of filing: 18.01.2017
(51) Int. Cl.: A61B 1/06, A61B 1/04, G02B 23/24, G02B 23/26

(54) **PROCESSOR FOR ENDOSCOPE**

(30) Priority: 20.01.2016 JP 2016008628
(71) Applicant: Olympus Corporation, Hachioji-shi Tokyo 192-8507 (JP)
(72) Inventor: SHIRAISHI Hiroshi, Hachioji-shi Tokyo 192-8507 (JP)
(74) Representative: Gunzelmann, Rainer
(86) International application number: PCT/JP2017/001489
(87) International publication number: WO 2017/126531

(57) **Abstract**

An endoscope processor is configured to control a light source device configured to supply illumination light for illuminating an object, an image of which is picked up by an endoscope including an insertion section insertable into a body cavity of a subject, and includes a halation detection unit configured to perform a process of detecting a generation state of halation in the image of the object; a light-adjusting unit configured to perform an operation of adjusting an amount of the illumination light supplied by the light source device to the endoscope, based on a processing result obtained by the process performed by the halation detection unit; and a control unit configured to select and set one light-adjusting mode matching a type of the endoscope categorized based on differences in a distal end structure of the insertion section, from among a plurality of light-adjusting modes specified by combinations of a process performed by the halation detection unit and an operation performed by the light-adjusting unit.

## Description

### Technical Field

The present invention relates to an endoscope processor, and more particularly, to an endoscope processor configured to adjust brightness of an observation image that is obtained at a time of endoscopic observation.

### Background Art

Various techniques that can be used at a time of adjusting brightness of an observation image that is obtained at the time of endoscopic observation to a desired brightness are conventionally known.

More specifically, for example, as a technique that can be used at the time of adjusting brightness of an entire region of an observation image that is obtained at the time of endoscopic observation to a certain brightness, Japanese Patent Publication No. 7-108278 discloses a technique of controlling an amount of illumination light that is radiated on an object, based on an average value of luminance when reflected light from the object is picked up.

Moreover, for example, as a technique that can be used at the time of adjusting brightness of a high-luminance region in an observation image that is obtained at the time of endoscopic observation to predetermined brightness, Japanese Patent Publication No. 7-108278 discloses a technique of controlling the amount of illumination light that is radiated on an object, based on a peak value of luminance when reflected light from the object is picked up.

For example, as a technique that can be used at the time of adjusting brightness of an observation image including a high-luminance object such as a treatment instrument and a high-luminance affected part such as angular incisure, Japanese Patent Application Laid-Open Publication No. 2004-321610 discloses a technique of performing setting such that halation at a surrounding part of the observation image is detected while detection of halation at a center portion of the observation image is suppressed as much as possible, as well as a technique of controlling the amount of illumination light by using a detection result of halation obtained according to such setting.

Generally, with endoscopic observation in a medical field, various types of endoscopes are used according to observation target parts in a body cavity or the like. Accordingly, with endoscopic observation in the medical field, light-adjusting control which is suitable for a type of endoscope used for observation is desirably performed.

However, neither Japanese Patent Publication No. 7-108278 nor Japanese Patent Application Laid-Open Publication No. 2004-321610 particularly discloses a method of controlling the amount of illumination light according to the type of endoscope used for observation. Therefore, the configurations disclosed in Japanese Patent Publication No. 7-108278 and Japanese Patent Application Laid-Open Publication No. 2004-321610 have a problem that brightness of an observation image that is obtained at the time of endoscopic observation cannot be adjusted to appropriate brightness according to a type of endoscope, for example.

The present invention has been made in view of the above circumstances, and aims to provide an endoscope processor which is capable of adjusting brightness of an observation image that is obtained at the time of endoscopic observation to appropriate brightness according to a type of endoscope.

### Disclosure of Invention

### Means for Solving the Problem

An endoscope processor according to an aspect of the present invention is an endoscope processor configured to control a light source device configured to supply illumination light for illuminating an object, an image of which is picked up by an endoscope including an insertion section insertable into a body cavity of a subject, the endoscope processor including a halation detection unit configured to perform a process of detecting a generation state of halation in the image of the object picked up by the endoscope; a light-adjusting unit configured to perform an operation of adjusting an amount of the illumination light supplied by the light source device to the endoscope, based on a processing result obtained by the process performed by the halation detection unit; and a control unit configured to perform an operation of selecting and setting one light-adjusting mode matching a type of the endoscope categorized based on differences in a distal end structure of the insertion section, from among a plurality of light-adjusting modes specified by combinations of a process performed by the halation detection unit and an operation performed by the light-adjusting unit.

### Brief Description of the Drawings

Fig. 1 is a diagram showing a configuration of main parts of an endoscopic system according to an embodiment;
Fig. 2 is a diagram for describing an example of a specific configuration of an image processing unit provided in a processor according to the embodiment;
Fig. 3 is a schematic diagram showing an example of an observation image that is displayed on a display device when an endoscope of type TA and a treatment instrument are used together;
Fig. 4 is a schematic diagram showing an example of an observation image that is displayed on the display device when an endoscope of type TB and a treatment instrument are used together;
Fig. 5 is a schematic diagram showing an example of an observation image that is displayed on the display device when an endoscope of type TC and a treatment instrument are used together; and
Fig. 6 is a schematic diagram showing an example of an observation image that is displayed on the display device when an endoscope of type TD and a treatment instrument are used together.

### Best Mode for Carrying Out the Invention

Hereinafter, an embodiment of the present invention will be described with reference to the drawings.

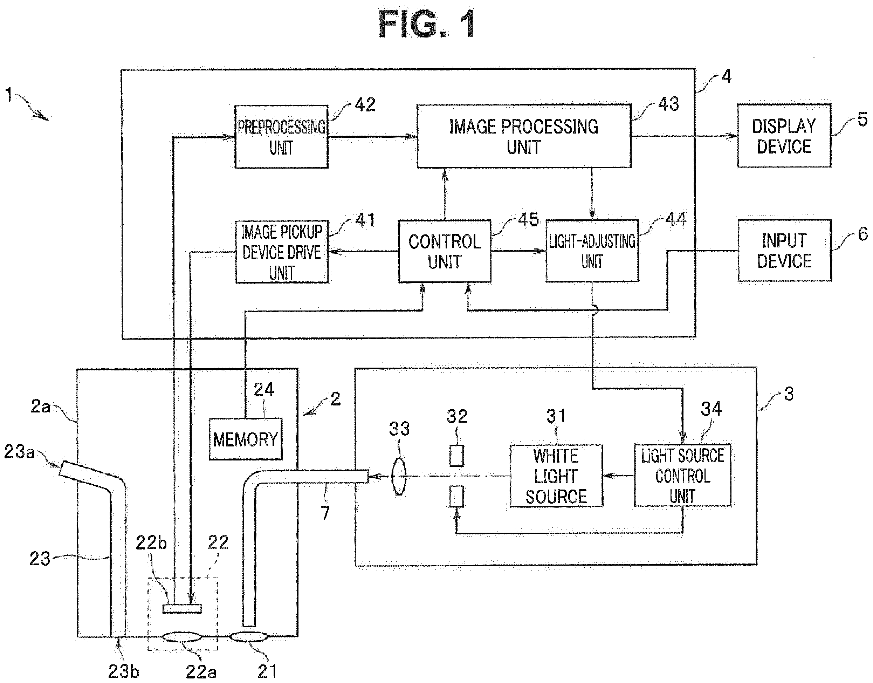

Figs. 1 to 6 are related to an embodiment of the present invention.

As shown in Fig. 1, an endoscopic system 1 is configured by including an endoscope 2 configured to be insertable into a body cavity of an examination subject, which is a living body, and to pick up an image of an object, such as a living body tissue, existing inside the body cavity and to output an image pickup signal, a light source device 3 configured to supply, to the endoscope 2, illumination light for illuminating the object, a processor 4 configured to generate and output an observation image which is according to the image pickup signal outputted from the endoscope 2, a display device 5 capable of displaying the observation image outputted from the processor 4, and the like, and an input device 6 capable of inputting, to the processor 4, information and/or an instruction according to an operation of a user, and the like. Fig. 1 is a diagram showing a configuration of main parts of the endoscopic system according to the embodiment.

The endoscope 2 is configured by including an insertion section 2a formed into an elongated shape so as to be insertable into a body cavity of an examination subject. Furthermore, the endoscope 2 is configured to be connectable to the processor 4 in a freely detachable manner by a scope connector, not shown. A nonvolatile memory 24 storing endoscope information including information unique to each endoscope 2 is provided in the endoscope 2.

A light guide 7 configured to transmit illumination light supplied by the light source device 3 to a distal end portion of the insertion section 2a is inserted inside the insertion section 2a. An illumination lens 21 configured to radiate illumination light emitted through the light guide 7 on an object, and an image pickup unit 22 configured to pick up reflected light (hereinafter referred to also as "return light") from the object illuminated with the illumination light and to output an image pickup signal are provided at the distal end portion of the insertion section 2a. A treatment instrument channel 23 allowing insertion of an elongated treatment instrument, which is used to treat a lesion or the like inside a body cavity of an examination subject, is provided inside the insertion section 2a.

The image pickup unit 22 is configured by including an objective lens 22a, and an image pickup device 22b.

The objective lens 22a is configured to form an optical image according to return light from an object illuminated with the illumination light that is emitted through the illumination lens 21.

For example, the image pickup device 22b is configured by including a CCD or a CMOS. Furthermore, the image pickup device 22b is configured by including a plurality of pixels for photoelectrically converting and picking up an optical image formed by the objective lens 22a, and a color filter provided on an image pickup surface, where the plurality of pixels are arranged two-dimensionally. Note that the color filter is formed by arranging, in a Bayer arrangement (in a checkered pattern), small filters of red (R), green (G), and blue (B) at positions corresponding to respective pixels of the image pickup device 22b, for example. Alternatively, the color filter may be formed by arranging small filters of yellow (Y), magenta (M), cyan (C), and G at positions corresponding to respective pixels of the image pickup device 22b, for example. The image pickup device 22b is configured to be driven according to an image pickup device drive signal outputted from the processor 4, to generate an image pickup signal by picking up an optical image formed by the objective lens 22a, and to output the generated image pickup signal to the processor 4.

The treatment instrument channel 23 is configured by including an insertion port 23a which is an opening provided at a proximal end portion of the insertion section 2a, and a projection port 23b which is an opening provided at the distal end portion of the insertion section 2a. The treatment instrument channel 23 is formed into a tubular shape allowing an elongated treatment instrument inserted from the insertion port 23a to project from the projection port 23b.

The memory 24 stores, as information unique to each endoscope 2, endoscope information including information indicating the type of the endoscope 2 and information indicating the number of pixels of the image pickup device 22b, for example. Note that in the present embodiment, a description is given assuming that types of the endoscopes 2 are categorized based on differences in layout of the illumination lens 21, the objective lens 22a, and the projection port 23b at the distal end portion of the insertion section 2a, or in other words, differences in a distal end structure of the insertion section 2a.

The light source device 3 is configured by including a white light source 31, a diaphragm device 32, a condenser lens 33, and a light source control unit 34.

The white light source 31 includes a xenon lamp or an LED, for example. Note that in the present embodiment, the white light source 31 may be configured to generate white light by combining lights generated by LEDs of three colors, R, G, and B, or may be configured to generate white light by combining lights generated by five LEDs of R, G, B, amber (A), and violet (V). The white light source 31 is also configured to be able to generate white light in an amount of light according to a light source control signal outputted from the light source control unit 34.

The diaphragm device 32 is provided on an optical path between the white light source 31 and the condenser lens 33. The diaphragm device 32 is configured to be able to increase or reduce the amount of white light emitted from the white light source 31 to the condenser lens 33, by changing an aperture value according to a light source control signal outputted from the light source control unit 34.

The condenser lens 33 is configured to condense white light emitted through the diaphragm device 32, and to emit the light to the light guide 7.

The light source control unit 34 is configured by including a light source control circuit, for example. Furthermore, the light source control unit 34 is configured to generate and output a light source control signal for controlling the white light source 31 and the diaphragm device 32, according to a light-adjusting signal outputted from the processor 4.

The processor 4 is configured by including an image pickup device drive unit 41, a preprocessing unit 42, an image processing unit 43, a light-adjusting unit 44, and a control unit 45.

The image pickup device drive unit 41 is configured by including a drive circuit for driving the image pickup device 22b, for example. The image pickup device drive unit 41 is configured to generate and output an image pickup device drive signal for driving the image pickup device 22b, according to a system control signal outputted from the control unit 45.

The preprocessing unit 42 is configured by including signal processing circuits such as a noise reduction circuit and an A/D conversion circuit, for example. The preprocessing unit 42 is configured to generate image data by subjecting the image pickup signal outputted from the endoscope 2 to signal processing such as noise reduction and A/D conversion, for example, and to sequentially output the generated image data frame by frame to the image processing unit 43, for each of R, G, and B. Note that in the present embodiment, in the case of generating image data by picking up an image of an object with an image pickup device provided with color filters of Y, M, C, and G, a field of odd-numbered lines and a field of even-numbered lines of image data of each color may be separately read.

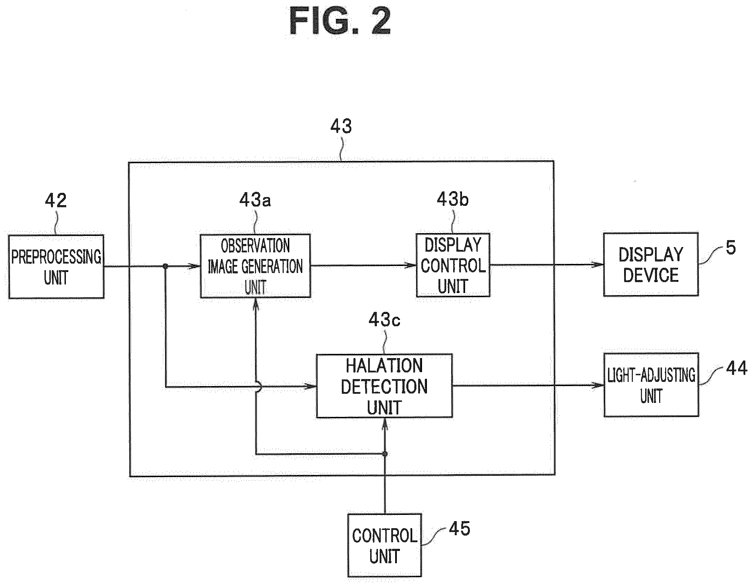

The image processing unit 43 is configured by including an image processing circuit, for example. The image processing unit 43 is configured to generate, according to a system control signal outputted from the control unit 45, a video signal based on image data which is sequentially outputted frame by frame from the preprocessing unit 42, and to output the generated video signal to the display device 5. Moreover, the image processing unit 43 is configured to perform, according to the system control signal outputted from the control unit 45, a process of detecting a generation state of halation in the image data which is sequentially outputted frame by frame from the preprocessing unit 42, and also to generate and output to the light-adjusting unit 44, a halation detection signal indicating a processing result obtained by the aforementioned process. More specifically, for example, the image processing unit 43 is configured by including an observation image generation unit 43a, a display control unit 43b, and a halation detection unit 43c, as shown in Fig. 2. Fig. 2 is a diagram for describing an example of a specific configuration of the image processing unit provided in the processor according to the embodiment.

The observation image generation unit 43a is configured to operate according to a system control signal outputted from the control unit 45. The observation image generation unit 43a is also configured to generate observation image data by subjecting the image data outputted from the preprocessing unit 42 to image processing such as gain adjustment and grayscale conversion, and to output the generated observation image data to the display control unit 43b.

The display control unit 43b is configured to generate a video signal by converting, according to predetermined video output standards, the observation image data outputted from the observation image generation unit 43a, and to output the generated video signal to the display device 5.

The halation detection unit 43c is configured to perform, according to a system control signal outputted from the control unit 45, a process of detecting a generation state of halation in image data outputted from the preprocessing unit 42, based on a luminance value of each pixel of the image data, and also to generate and output to the light-adjusting unit 44, a halation detection signal indicating a processing result obtained by the aforementioned process.

The light-adjusting unit 44 is configured by including a light-adjusting circuit, for example. The light-adjusting unit 44 is configured to generate, and output to the light source control unit 34, a light-adjusting signal for causing a predetermined amount of white light to be generated by the white light source 31, when power of the processor 4 is activated and the light source device 3 is connected to the processor 4. Furthermore, the light-adjusting unit 44 is configured to generate, and output to the light source control unit 34, a light-adjusting signal for adjusting the amount of illumination light supplied by the light source device 3 to the endoscope 2 by a change in the aperture value of the diaphragm device 32, based on a halation detection signal outputted from the image processing unit 43 and a system control signal outputted from the control unit 45.

The control unit 45 is configured by including an electronic circuit, such as a CPU or a field programmable gate array (FPGA), which operates according to a predetermined program, for example.

The control unit 45 is configured to generate, and output to the image pickup device drive unit 41, a system control signal for controlling exposure time and the like of the image pickup device 22b.

The control unit 45 is configured to perform an operation of reading the endoscope information from the memory 24, when the power of the processor 4 is activated and the endoscope 2 is connected to the processor 4. The control unit 45 is also configured to set a light measurement region, which is a region corresponding to a detection region when detecting generation of halation, according to the number of pixels of the image pickup device 22b identified based on the endoscope information read from the memory 24, and to generate, and output to the halation detection unit 43c, a system control signal indicating the light measurement region that is set. Note that in the present embodiment, for the sake of simplicity, a description is given assuming that a light measurement region having a size and shape matching the image data outputted from the preprocessing unit 42 is set (according to the number of pixels of the image pickup device 22b).

The control unit 45 is configured to generate, upon detection of an instruction to manually set a light-adjusting mode of the processor 4, which is issued by a light-adjusting mode setting switch (not shown) of the input device 6, a system control signal for causing an operation to be performed according to the light-adjusting mode set in response to the operation of the input device 6, and to output the system control signal to the halation detection unit 43c and the light-adjusting unit 44.

Upon detection of an instruction to automatically set the light-adjusting mode of the processor 4, which is issued by the light-adjusting mode setting switch of the input device 6, the control unit 45 invalidates the light-adjusting mode set in response to operation of the input device 6, sets the light-adjusting mode matching the type of the endoscope 2 identified based on the endoscope information read from the memory 24, and generates a system control signal for causing an operation to be performed according to the set light-adjusting mode and outputs the system control signal to the halation detection unit 43c and the light-adjusting unit 44. In other words, the control unit 45 is configured to perform an operation of selecting and setting one light-adjusting mode matching the type of the endoscope 2 categorized based on differences in the distal end structure of the insertion section 2a, from among a plurality of light-adjusting modes specified by combinations of a process performed by the halation detection unit 43c and an operation performed by the light-adjusting unit 44.

The display device 5 includes a liquid crystal display (LCD) or the like, and is configured to be able to display, on a display screen, an observation image or the like according to a video signal outputted from the processor 4.

The input device 6 is configured by including a keyboard, a touch panel and/or a foot switch, for example. Note that the input device 6 may be a device separate from the processor 4, or an interface integrated with the processor 4, or an interface integrated with the endoscope 2. Moreover, the input device 6 is provided with the light-adjusting mode setting switch allowing selection of either manually or automatically to set the light-adjusting mode of the processor 4, and allowing selection of the light-adjusting mode of the processor 4 at a time of manual setting, from among a plurality of light-adjusting modes exemplified by first to third light-adjusting modes described later.

Next, specific operation and the like of the endoscopic system 1 will be described.

After connecting each unit of the endoscopic system 1 and turning on the power, a user such as a surgeon operates the light-adjusting mode setting switch of the input device 6 to issue an instruction to automatically set the light-adjusting mode of the processor 4.

The control unit 45 sets a light measurement region according to the number of pixels of the image pickup device 22b identified based on the endoscope information read from the memory 24, and generates, and outputs to the halation detection unit 43c, a system control signal indicating the set light measurement region.

According to the system control signal outputted from the control unit 45, the halation detection unit 43c performs a process of matching a center of the light measurement region indicated by the system control signal and a center of image data outputted from the preprocessing unit 42.

When an instruction to automatically set the light-adjusting mode of the processor 4 is detected, the control unit 45 sets the light-adjusting mode matching the type of the endoscope 2 identified based on the endoscope information read from the memory 24, and generates a system control signal for causing an operation to be performed according to the set light-adjusting mode and outputs the system control signal to the halation detection unit 43c and the light-adjusting unit 44.

Specific operation of the halation detection unit 43c, the light-adjusting unit 44, and the control unit 45 will now be described. Note that in the following, a description will be given citing as an example a case where the control unit 45 selects and sets one light-adjusting mode matching the type of the endoscope 2, from among three light-adjusting modes of a first light-adjusting mode, a second light-adjusting mode, and a third light-adjusting mode.

First, operation and the like of the halation detection unit 43c, the light-adjusting unit 44, and the control unit 45 in the first light-adjusting mode will be described.

After setting the light-adjusting mode to the first light-adjusting mode, the control unit 45 causes the halation detection unit 43c to perform a process of mainly detecting halation generated at a center portion of image data of one frame outputted from the preprocessing unit 42 rather than halation generated at a peripheral portion of the image data, and also generates and outputs a system control signal for causing the light-adjusting unit 44 to perform an operation of reducing the amount of illumination light supplied by the light source device 3 according to an increase in a generated amount of halation obtained as a processing result of the aforementioned process.

When the first light-adjusting mode is set, the halation detection unit 43c divides image data of one frame outputted from the preprocessing unit 42 into two regions of the center portion and the peripheral portion, detects a halation region Ac at the center portion based on a luminance value of each pixel belonging to the center portion, and detects a halation region Ap at the peripheral portion based on a luminance value of each pixel belonging to the peripheral portion. Then, the halation detection unit 43c performs a process of calculating a ratio Rc of the number of pixels in the halation region Ac to a total number of pixels in the center portion of the image data of the one frame outputted from the preprocessing unit 42, and of calculating a ratio Rp of the number of pixels in the halation region Ap to a total number of pixels in the peripheral portion of the image data. Then, the halation detection unit 43c generates, and outputs to the light-adjusting unit 44, a halation detection signal indicating a calculated value CV obtained by adding the ratio Rp to a value obtained by multiplying the ratio Rc by a coefficient α which takes a value of one or more (i.e., by performing calculation corresponding to α × Rc + Rp). Note that the coefficient α is a variable value that is set according to the ratio Rc, for example.

When the first light-adjusting mode is set, the light-adjusting unit 44 generates a light-adjusting signal for adjusting the amount of illumination light supplied by the light source device 3, based on the calculated value CV indicated by the halation detection signal outputted from the halation detection unit 43c, and outputs the light-adjusting signal to the light source control unit 34. More specifically, when the first light-adjusting mode is set, the light-adjusting unit 44 generates, and outputs to the light source control unit 34, a light-adjusting signal for increasing the aperture value of the diaphragm device 32 according to an increase in the calculated value CV, and for reducing the aperture value of the diaphragm device 32 according to a reduction in the calculated value CV, for example.

That is, when the operation as described above is performed in the first light-adjusting mode, generation of halation in the center portion of an observation image displayed on the display device 5 may be therefore suppressed while maintaining brightness of the entire observation image.

Next, operation and the like of the halation detection unit 43c, the light-adjusting unit 44, and the control unit 45 in the second light-adjusting mode will be described.

After setting the light-adjusting mode to the second light-adjusting mode, the control unit 45 causes the halation detection unit 43c to perform a process of detecting a generation state of halation in an entire region of an image of one frame outputted from the preprocessing unit 42, and also generates and outputs a system control signal for causing the light-adjusting unit 44 to perform an operation of reducing the amount of illumination light supplied by the light source device 3 according to an increase in a generated amount of halation obtained as a processing result of the aforementioned process.

When the second light-adjusting mode is set, the halation detection unit 43c detects a halation region Ah1 included in image data of one frame outputted from the preprocessing unit 42, based on a luminance value of each pixel of the image data. Then, the halation detection unit 43c performs a process of calculating a ratio Rh1 of the number of pixels in the halation region Ah1 to a total number of pixels of the image data of the one frame outputted from the preprocessing unit 42. Then, the halation detection unit 43c generates, and outputs to the light-adjusting unit 44, a halation detection signal indicating the ratio Rh1.

When the second light-adjusting mode is set, the light-adjusting unit 44 generates a light-adjusting signal for adjusting the amount of illumination light supplied by the light source device 3, based on the ratio Rh1 indicated by the halation detection signal outputted from the halation detection unit 43c, and outputs the light-adjusting signal to the light source control unit 34. More specifically, when the second light-adjusting mode is set, the light-adjusting unit 44 generates, and outputs to the light source control unit 34, a light-adjusting signal for increasing the aperture value of the diaphragm device 32 according to an increase in the ratio Rh1, and for reducing the aperture value of the diaphragm device 32 according to a reduction in the ratio Rh1, for example.

That is, when the operation as described above is performed in the second light-adjusting mode, generation of halation in an observation image displayed on the display device 5 may be therefore suppressed while preventing reduction in brightness of a high-luminance region in the observation image as much as possible.

Lastly, operation and the like of the halation detection unit 43c, the light-adjusting unit 44, and the control unit 45 in the third light-adjusting mode will be described.

After setting the light-adjusting mode to the third light-adjusting mode, the control unit 45 causes the halation detection unit 43c to perform a process of detecting a generation state of halation in an entire region of an image of one frame outputted from the preprocessing unit 42, and also generates and outputs a system control signal for causing the light-adjusting unit 44 to perform an operation of maintaining the amount of illumination light supplied by the light source device 3, in a case where a generated amount of halation obtained as a processing result of the aforementioned process, is less than a predetermined amount, and an operation of reducing the amount of illumination light, in a case where the generated amount of halation obtained as the processing result of the aforementioned process, is equal to or greater than the predetermined amount.

When the third light-adjusting mode is set, the halation detection unit 43c detects a halation region Ah2 included in image data of one frame outputted from the preprocessing unit 42, based on a luminance value of each pixel of the image data. Then, the halation detection unit 43c performs a process of calculating a ratio Rh2 of the number of pixels in the halation region Ah2 to a total number of pixels of the image data of the one frame outputted from the preprocessing unit 42. Then, the halation detection unit 43c generates, and outputs to the light-adjusting unit 44, a halation detection signal indicating the ratio Rh2.

When the third light-adjusting mode is set, the light-adjusting unit 44 generates a light-adjusting signal for adjusting the amount of illumination light supplied by the light source device 3, based on the ratio Rh2 indicated by the halation detection signal outputted from the halation detection unit 43c, and outputs the light-adjusting signal to the light source control unit 34. More specifically, when the third light-adjusting mode is set, the light-adjusting unit 44 generates, and outputs to the light source control unit 34, a light-adjusting signal for maintaining the aperture value of the diaphragm device 32 at a predetermined value in a case where the ratio Rh2 is below a threshold TH, and for increasing the aperture value of the diaphragm device 32 to a value equal to or greater than the predetermined value in a case where the ratio Rh2 is equal to or greater than the threshold TH.

That is, when the operation as described above is performed in the third light-adjusting mode, generation of halation in an observation image displayed on the display device 5 may be therefore suppressed as much as possible while stabilizing brightness of the entire observation image.

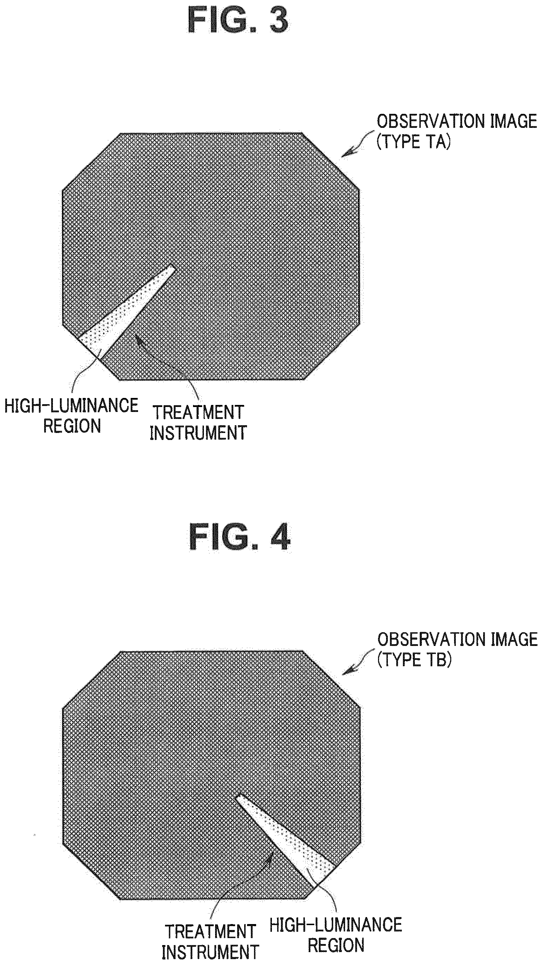

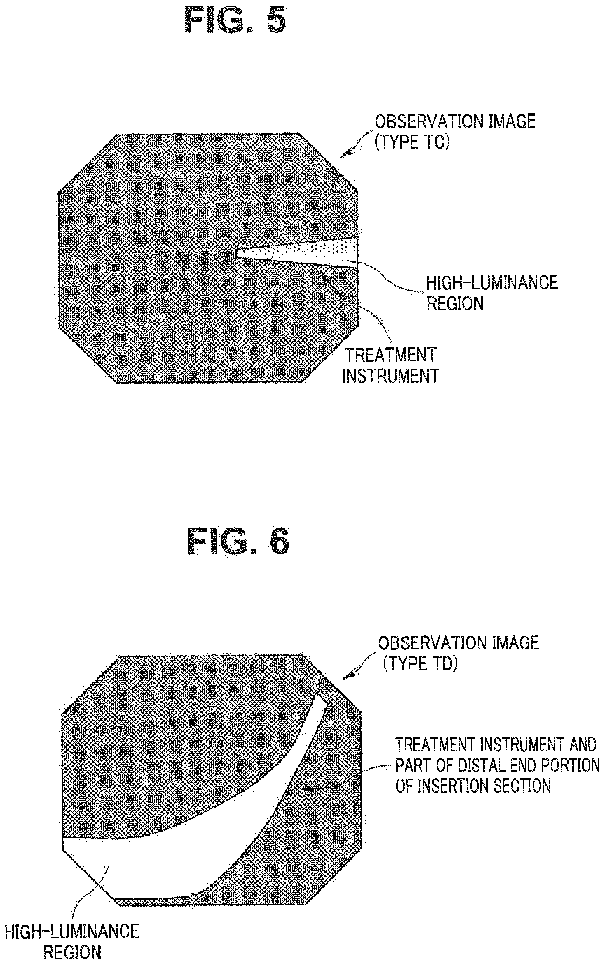

With an observation image which is displayed on the display device 5 when the endoscope 2 and a treatment instrument are used together, a phenomenon may occur according to which a position and/or an area of a high-luminance region where halation is possibly generated due to radiation of illumination light on the treatment instrument projected from the projection port 23b are different for each type of endoscope 2, as schematically shown in Figs. 3 to 5, for example. Note that type TA in Fig. 3 is one of an upper digestive tract endoscope, a lower digestive tract endoscope, a bronchial endoscope, a nasopharyngeal endoscope, a bladder endoscope, and the like. Type TB in Fig. 4 is one of the upper digestive tract endoscope, the lower digestive tract endoscope, the bronchial endoscope, the nasopharyngeal endoscope, the bladder endoscope, and the like, which is different from the type TA. Type TC in Fig. 5 is one of the upper digestive tract endoscope, the lower digestive tract endoscope, the bronchial endoscope, the nasopharyngeal endoscope, the bladder endoscope, and the like, which is different from either the type TA or the type TB. Fig. 3 is a schematic diagram showing an example of an observation image that is displayed on the display device when an endoscope of the type TA and a treatment instrument are used together. Fig. 4 is a schematic diagram showing an example of an observation image that is displayed on the display device when an endoscope of the type TB and a treatment instrument are used together. Fig. 5 is a schematic diagram showing an example of an observation image that is displayed on the display device when an endoscope of the type TC and a treatment instrument are used together.

In the present embodiment, with respect to occurrence of the phenomenon described above, the control unit 45 selects one light-adjusting mode matching the type of the endoscope 2, from among the plurality of light-adjusting modes exemplified by the first to the third light-adjusting modes, and performs control of causing the halation detection unit 43c and the light-adjusting unit 44 to operate. Accordingly, in the present embodiment, brightness of an observation image that is displayed on the display device 5, or in other words, brightness of an observation image that is obtained at the time of endoscopic observation, may be adjusted to appropriate brightness according to the type of the endoscope 2.

When the endoscope 2 of type TD having a distal end structured to include a treatment instrument projected from the projection port 23b at the distal end portion of the insertion section 2a and a part of the distal end portion in an emission range of illumination light that is emitted through the illumination lens 21 is connected to the processor 4, the control unit 45 of the present embodiment selects the third light-adjusting mode from among the three light-adjusting modes described above.

For example, as schematically shown in Fig. 6, with an observation image that is displayed on the display device 5 when the endoscope 2 of the type TD and a treatment instrument are used together, the position and area of the high-luminance region are greatly related to change in brightness of the entire observation image. Accordingly, in the present embodiment, when the endoscope 2 of the type TD is connected to the processor 4, the halation detection unit 43c and the light-adjusting unit 44 perform operation according to the third light-adjusting mode so that brightness of the entire observation image displayed on the display device 5 may be prevented as much as possible from being changed, even in a case where the position and area of the high-luminance region are frequently changed, for example. Note that the type TD in Fig. 6 is a duodenum endoscope, for example, and is, thus, a type different from any of the type TA, the type TB, and the type TC. Fig. 6 is a schematic diagram showing an example of an observation image that is displayed on the display device when the endoscope of the type TD and a treatment instrument are used together.

Note that the present invention is not limited to the embodiment described above, and it is needless to say that various modifications and applications are allowed without departing from the gist of the invention.

The present application claims priority from Japanese Patent Application No. 2016-008628 filed in Japan on January 20, 2016, the entire contents of which are incorporated herein by reference.

## Claims

1. An endoscope processor configured to control a light source device configured to supply illumination light for illuminating an object, an image of which is picked up by an endoscope including an insertion section insertable into a body cavity of a subject, the endoscope processor comprising:
a halation detection unit configured to perform a process of detecting a generation state of halation in the image of the object picked up by the endoscope;
a light-adjusting unit configured to perform an operation of adjusting an amount of the illumination light supplied by the light source device to the endoscope, based on a processing result obtained by the process performed by the halation detection unit; and
a control unit configured to perform an operation of selecting and setting one light-adjusting mode matching a type of the endoscope categorized based on differences in a distal end structure of the insertion section, from among a plurality of light-adjusting modes specified by combinations of a process performed by the halation detection unit and an operation performed by the light-adjusting unit.

2. The endoscope processor according to claim 1, wherein the plurality of light-adjusting modes include
a first light-adjusting mode of causing the halation detection unit to perform a first process of mainly detecting halation generated at a center portion of the image rather than halation generated at a peripheral portion of the image, and also of causing the light-adjusting unit to perform an operation of reducing an amount of the illumination light according to an increase in a generated amount of halation obtained as a processing result of the first process,
a second light-adjusting mode of causing the halation detection unit to perform a second process of detecting a generation state of halation in an entire region of the image, and also of causing the light-adjusting unit to perform an operation of reducing an amount of the illumination light according to an increase in a generated amount of halation obtained as a processing result of the second process, and
a third light-adjusting mode of causing the halation detection unit to perform the second process, and also of causing the light-adjusting unit to perform an operation of maintaining an amount of the illumination light in a case where a generated amount of halation obtained as a processing result of the second process is less than a predetermined amount, and an operation of reducing the amount of the illumination light in a case where the generated amount of halation obtained as the processing result of the second process is equal to or greater than the predetermined amount.

3. The endoscope processor according to claim 2, wherein the control unit performs an operation of selecting and setting the third light-adjusting mode in a case where the type of the endoscope is a predetermined type including a distal end structure to include a treatment instrument projected from a distal end portion of the insertion section and a part of the distal end portion in an emission range of the illumination light emitted through the distal end portion.

4. The endoscope processor according to claim 3, wherein the predetermined type is a duodenum endoscope.
